# EUROPEAN PATENT APPLICATION

(11) **EP 4 846 993 A1**
(43) Date of publication of application: **02.12.2026**
(21) Application number: 25202022.7
(22) Date of filing: 12.09.2025
(51) Int. Cl.: A61F 13/494

(54) **ABSORBENT ARTICLE WITH IMPROVED BARRIER**

(30) Priority: 30.05.2025 EP 25179990; 30.05.2025 EP 25180003; 24.06.2025 EP 25185053
(71) Applicant: Ontex BV, 9255 Buggenhout (BE); Ontex Group NV, 9320 Erembodegem (BE)
(72) Inventor: Van Sande, Nathalie, 9688 Maarkedal (BE)
(74) Representative: Cobbaert, Dries

(57) **Abstract**

An absorbent article includes a liquid permeable topsheet, a liquid impermeable backsheet, and an absorbent core. Barrier leg cuffs are formed from a single web of material, each having an inner cuff element with elastic elements and an outer cuff element with outer elastic elements. The outer elastic elements are disposed between parts of the liquid impermeable backsheet. This configuration improves leakage protection with a simplified cuff design.

## Description

### TECHNICAL FIELD

The present invention relates to absorbent articles, such as diapers, training pants, and incontinence products, having barrier leg cuffs and a liquid impermeable backsheet.

### BACKGROUND

Absorbent articles, such as diapers, are designed to absorb and contain bodily exudates. A common challenge in absorbent article design is preventing side leakage, especially when the article becomes saturated or during sudden gushes of fluid. To address this, manufacturers often incorporate barrier leg cuffs with elastic gathering elements along the lateral edges. These cuffs are meant to create a seal against the wearer's skin, preventing fluids from escaping the article.

WO202408297 describes an absorbent article having a nonwoven outer cover layer which overlies the backsheet and forms an outer surface of the absorbent article and which has side portions which extend laterally outside the backsheet and are formed into leg cuff folds, the leg cuff folds being elasticated by a plurality of spaced-apart elastic elements. A pair of nonwoven barrier cuff strips are arranged with a standing gather portion of each barrier cuff strip forming part of a standing gather, the barrier cuff strip being attached to the topsheet along a standing gather hinge line and having one or more pre-tensioned elastic elements arranged along a free standing gather edge. A pair of leg cuffs are arranged outward of the standing gathers, each leg cuff comprising the folded-back, elasticated side portion of the outer cover layer and a leg cuff portion of the corresponding nonwoven barrier strip. The leg cuff portion of the nonwoven barrier strip overlies at least one elastic element of the plurality of elastic elements.

EP 2 992 864 B1 discloses an absorbent article for personal hygiene such as a diaper or training pant having a front edge and a back edge. The article has a length L as measured along the longitudinal axis from its front edge to its back edge, and a crotch point (C) defined as the point placed at a distance of two fifth of L from the front edge of the article on the longitudinal axis. The article comprises a liquid permeable topsheet, a liquid impermeable backsheet, a pair of barrier leg cuffs extending at least partially between the front edge and the back edge of the diaper on opposite sides of the longitudinal axis and present at least at the longitudinal position of the crotch point, each barrier leg cuff being delimited by a proximal edge joined directly or indirectly to the topsheet and/or the backsheet and a free terminal edge, and an absorbent core comprising a core wrap enclosing an absorbent material, wherein the absorbent material comprises at least 80% of superabsorbent polymers by weight of the absorbent material, wherein the absorbent core comprises at least one channel at least partially oriented in the longitudinal direction of the article.

There remains a need in the art for absorbent articles with improved leakage protection, simplified construction, enhanced comfort, and minimized manufacturing costs. It is desirable to further improve the effectiveness of leg cuffs while reducing complexity and optimizing material usage.

### SUMMARY

According to a first aspect of the disclosure, an absorbent article comprises: a liquid permeable topsheet; a liquid impermeable backsheet; an absorbent core disposed at least partially between the topsheet and the backsheet; and a pair of barrier leg cuffs, each barrier leg cuff formed from a single web of material and comprising an inner cuff element and an outer cuff element; wherein the inner cuff element comprises an inner folded edge and one or more, preferably a plurality of, inner elastic elements, wherein the inner elastic elements are disposed proximate the inner folded edge; and wherein the outer cuff element comprises an outer folded edge and from 1 to 5 outer elastic elements; characterised in that the outer elastic elements are disposed proximate the outer folded edge; and in that the one or more outer elastic elements are disposed at least partly between a first part and a second part of said liquid impermeable backsheet. This configuration may provide an effective barrier against side leakage by combining the single web construction of the cuffs with a specific backsheet arrangement.

According to a preferred embodiment of the disclosure, an absorbent article comprises an inner cuff element forming an up-standing gather. This may enhance the containment of fluids within the absorbent article.

According to a preferred embodiment of the disclosure, an absorbent article comprises a longitudinal center axis and an inner cuff element substantially T-shaped, preferably when viewed from a section taken about an axis being substantially perpendicular to the longitudinal center axis. The T-shape may provide a balance of sealing and comfort.

According to a preferred embodiment of the disclosure, an absorbent article has a liquid impermeable backsheet comprising an outer folded edge such that the first part of said backsheet is folded laterally inward towards the longitudinal centerline in overlapping configuration with said second part of said backsheet. This configuration may provide additional leakage protection and structural integrity.

According to a preferred embodiment of the disclosure, an absorbent article is provided wherein said outer elastic elements are disposed proximal the outer folded edge of said liquid impermeable backsheet. Positioning the outer elastic elements near the folded edge may enhance the seal against the wearer's body.

According to a preferred embodiment of the disclosure, an absorbent article is provided wherein said absorbent core comprises 2 laterally opposed longitudinal side edges, a transversal front edge and a transversal back edge and wherein said first part of said liquid impermeable backsheet extends along the entire thickness of the absorbent core. This may ensure that the backsheet fully protects the absorbent core from leakage.

According to a preferred embodiment of the disclosure, an absorbent article is provided wherein said absorbent core comprises a top core wrap layer and a bottom core wrap layer and absorbent material arranged partially between said top core wrap layer and said bottom core wrap layer. This may provide a structured absorbent core with distinct layers for different functions.

According to a preferred embodiment of the disclosure, an absorbent article is provided wherein the absorbent core has a maximum width mW1, the top core wrap layer has a maximum width mW2 and the bottom core wrap layer has a maximum width mW3, wherein mW1 is greater than mW2 and/or mW3. This configuration may optimize the absorbent capacity and fluid distribution within the core.

According to a preferred embodiment of the disclosure, an absorbent article is provided wherein said first part of said liquid impermeable backsheet is attached to the wearer-facing surface of the absorbent core. This may provide a secure and stable construction.

According to a preferred embodiment of the disclosure, an absorbent article is provided wherein said first part of said liquid impermeable backsheet comprises an end edge and wherein said end edge is attached to the wearer-facing surface of the absorbent core. This may further stabilize the backsheet and may prevent shifting.

According to a preferred embodiment of the disclosure, an absorbent article is provided wherein said first part of said liquid impermeable backsheet comprises an end edge and wherein said end edge is attached to the wearer-facing side and/or the garment-facing side of top core wrap layer. This may provide a secure and stable construction.

According to a preferred embodiment of the disclosure, an absorbent article wherein said first part of said liquid impermeable backsheet is attached to the garment-facing surface of the absorbent core. This simplifies the construction and secures the backsheet to the core. According to a preferred embodiment of the disclosure, an absorbent article wherein said first part of said liquid impermeable backsheet comprises an end edge and wherein said end edge is attached to the garment-facing surface of the absorbent core. This connection further secures the backsheet to the core and prevents leakage.

According to a preferred embodiment of the disclosure, an absorbent article is provided wherein said first part of said liquid impermeable backsheet comprises an end edge and wherein said end edge is attached to the wearer-facing surface of the bottom core wrap layer and/or to the garment-facing surface of the bottom core wrap layer. This may provide a secure and stable construction.

According to a preferred embodiment of the disclosure, an absorbent article is provided wherein said absorbent article further comprises a NW backsheet at least partially attached to the garment-facing surface of said liquid impermeable backsheet. The NW backsheet may provide a soft and cloth-like outer covering.

According to a preferred embodiment of the disclosure, an absorbent article is provided wherein said NW backsheet is attached to the liquid impermeable backsheet by ultrasonic bonding. This may create a strong and reliable bond between the layers.

According to a preferred embodiment of the disclosure, an absorbent article is provided wherein said single web of material comprises an end edge and said end edge is attached to the garment-facing surface of the NW backsheet. This may secure the single web material to the outer layers of the article.

According to a preferred embodiment of the disclosure, an absorbent article is provided wherein said single web of material comprises an end edge and said end edge is attached to the wearer-facing surface of the NW backsheet. This may secure the single web material to the inner layers of the article.

According to a preferred embodiment of the disclosure, an absorbent article is provided wherein said article comprises a transverse center axis perpendicular to the longitudinal center axis and wherein said NW backsheet has a length L1 parallel to the longitudinal center axis and said liquid impermeable backsheet has a length L2 parallel to the longitudinal center axis, wherein L1 is less than L2. This may optimize the coverage and functionality of the NW backsheet.

### BRIEF DESCRIPTION OF THE FIGURES

The invention can be better understood with reference to the following drawings and description. The components in the figures are not necessarily to scale, emphasis instead being placed upon illustrating various preferred embodiments of the invention.
Fig. 1 is a top plan view of an absorbent article according to an aspect of the disclosure.
Fig. 2 is a cross-section view of an absorbent article about the transverse center line x-x.
Fig. 3 is a cross-section view of an absorbent article about the transverse center line x-x, showing an exemplary standing-up gather.
Fig. 4 is a cross-section view of an absorbent article (several components have been removed for legibility) about the transverse center line x-x, showing the subdivision of the liquid impermeable backsheet.
Fig. 5 is a cross-section view of an absorbent article (several components have been removed for legibility) about the transverse center line x-x, showing an example of difference in width between the absorbent core and the core wrap layers.
Fig. 6A-6C are cross-section views of an absorbent article about the transverse center line x-x, showing different embodiments of the present disclosure.
Fig.7A-7C are cross-section views of an absorbent article about the transverse center line x-x, showing different embodiments of the present disclosure.
Fig.8-9 are cross-section views of an absorbent article about the transverse center line x-x, showing embodiments of the absorbent article comprising a NW backsheet.
Fig. 10 is a cross-section view of an absorbent article about the longitudinal center line y-y according to the present disclosure.

### DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
The expression "% by weight" (weight percent) otherwise referred to as "wt%", here and throughout the description unless otherwise defined, refers to the relative weight of the respective component based on the overall weight of the formulation.

The recitation of numerical ranges by endpoints includes all numbers and fractions subsumed within that range, as well as the recited endpoints unless otherwise stated.

"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. **It** is common to combine cellulosic fluff pulp with superabsorbent polymers in an absorbent material.

"Acquisition and distribution layer", "ADL" or "surge management portion" refers to a sublayer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, an "airformed web" refers to a material comprising cellulosic fibers such as those from fluff pulp that have been separated, such as by a hammermilling process, and then deposited on a porous surface without a substantial quantity of binder fibers present. Airfelt materials used as the absorbent core in many diapers, for example, are a typical example of an airformed material.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present invention can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present invention. Binder can be added to an airformed web in liquid form (e. g., an aqueous solution or a melt) by spray nozzles, direction injection or impregnation, vacuum drawing, foam impregnation, and so forth. Solid binder particles can also be added by mechanical or pneumatic means.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The term "bio-based" may refer to a component of an absorbent article that can be produced or is derived from a renewable resource, as defined herein.

As used herein, the "body-facing" or "bodyside" or "wearer-facing" surface means that surface of the article or component which is intended to be disposed toward or placed adjacent to the body of the wearer during ordinary use, while the "outward", "outward-facing", "garment-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to layers, preferably films or elastic laminates, having a water vapor transmission rate (WVTR) of at least 300 grams/m² - 24 hours.

"Carded web" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

As used herein "channels" are fluid distribution means within the absorbent core adapted to favour exudate flow there along and are typically intended to exclude embossing patterns or ducts formed by compression from the meaning thereof and rather include structures that are substantially free of absorbent material instead of comprising compacted absorbent material. Channels herein are formed by joining upper and lower layers of a core wrap as will be described in more detail hereinbelow. Channels preferably comprise recessed regions forming visible conduits or passages typically extending along the longitudinal axis of the core and having a depth in a direction perpendicular to said longitudinal axis. By "visible" it is herein intended clearly visible by naked eye. Typically the channels have a width generally greater than 1mm, preferably from 5mm to 50mm, more preferably from 6mm to 40mm, more preferably from 8mm to 30mm, even more preferably from greater than 8mm to less than 25mm.

"Chassis" refers to a foundational constituent of an absorbent article upon which the remainder of the structure of the article is built up or overlaid, e.g., in a diaper, the structural elements that give the diaper the form of briefs or pants when configured for wearing, such as a back sheet, a top sheet, or a combination of a top sheet and a back sheet.

"Comprise", "comprising", and "comprises" and "comprised of" as used herein are synonymous with "include", "including", "includes" or "contain", "containing", "contains" and are inclusive or openended terms that specifies the presence of what follows e.g. component and do not exclude or preclude the presence of additional, non-recited components, features, element, members, steps, known in the art or disclosed therein.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

"Continuous" means that the described structure is a closed-loop structure. The continuous structure may be unitary, i.e., a one-piece structure, or may be made up of individual elements suitably joined together to form a closed-loop.

"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

"Decitex" also known as Dtex is a measurement used in the textile industry for measuring yarns or filaments. 1 Decitex = 1 gram per 10,000 meters. In other words, if 10,000 linear meters of a relaxed yarn or filament weights 500 grams that yarn or filament would have a decitex of 500.

The term "density" or "concentration" when referring to the absorbent material, in particular the SAP, of a layer, refers to the amount of the absorbent material divided by the surface area of the layer over which the absorbent material is spread out.

"Discontinuous bonding pattern" as used herein refers to a pattern of bonding areas, in particular bonding areas between layers, whereby at least in at least one region the layers are not bonded. A discontinuous bonding pattern may comprise a connected bonding area or multiple disconnected bonding areas. A discontinuous bonding pattern further may comprise a connected bonding area comprising a number of holes, where the layers are not bonded, preferably according to a regular pattern, or it may comprise discrete disconnected bonding areas, e.g. a point bonded pattern which comprises a plurality of separate bonding points surrounded by unbonded areas or a line-bonded pattern which comprises a plurality of separate bonding lines alternated by unbonded areas, preferably according to a regular pattern.

The term "disposable" may be used herein to describe absorbent articles and other products which are not intended to be laundered or otherwise restored or reused as an absorbent article or product (i.e., they are intended to be discarded after use and, preferably, to be recycled, composted or otherwise disposed of in an environmentally compatible manner).

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" or "elastified" refers to a material, layer, or substrate that is naturally non-elastic, but which has been rendered elastic by, for example, suitably joining an elastic material, layer, or substrate thereto.

As used herein, the term "elastic resistance" describes an elastic force that tends to resist an applied tensile force causing a material provided therewith to tend to contract to an untensioned configuration in response to a stretching force.

"Elongation" means the ratio of the extension of a material to the length of the material prior to the extension (expressed in percent). "Extension" means the change in length of a material due to stretching (expressed in units of length).

As used herein the term "extensible" means elongatable in at least one direction, but not necessarily recoverable.

The term "film" refers to a sheet-like material wherein the length and width of the material far exceed the thickness of the material. Typically, films have a thickness of about 0.5 mm or less.

The term "flexible" refers to materials which are compliant and which will readily conform to the general shape and contours of the wearer's body.

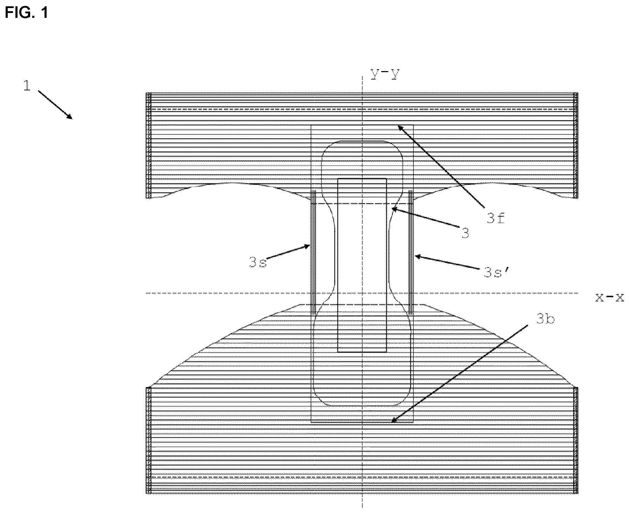

The term "fully extended state" refers to the state in which the absorbent article is laid flat, with all elastic components extended to the point where the material in which they are incorporated is maximally extended in the direction the materials are designed to elongate when in use, but without causing damage or permanent deformation to any of the article's components. For the purpose of dimensional measurements, the "fully extended state" may correspond to a 2D technical drawing, prepared by a designer for production purposes, showing a top plan view of the article in an opened-up and laid-flat state, with all elastic components extended as described above and as shown for example in Figure 1. **In** case of discrepancy, the 2D technical drawing takes precedence over the physical extension of the article.

The term "high-absorbency material" refers to materials that are capable of absorbing at least 10 times their own weight in liquid. The high-absorbency material may comprise absorbent gelling materials, such as superabsorbent polymers. Superabsorbent polymers are water-swellable, water-insoluble organic or inorganic materials capable of absorbing at least about 20 times their own weight of an aqueous solution containing 0.9 weight percent of sodium chloride. Absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces. Examples of synthetic absorbent gelling material polymers include the alkali metal and ammonium salts of poly(acrylic acid) and poly (methacrylic acid), poly(acrylamides), poly(vinyl ethers), maleic anhydride copolymers with vinyl ethers and alphaolefins, poly(vinyl pyrrolidone), poly(vinylmorpholinone), poly(vinyl alcohol), and mixtures and copolymers thereof. Further polymers suitable for use in the absorbent structure include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum and the like. Mixtures of natural and wholly or partially synthetic absorbent polymers can also be used. Synthetic absorbent gelling materials typically are xerogels which form hydrogels when wetted. The term "hydrogel", however, has commonly been used to also refer to both the wetted and unwetted forms of the material. The high-absorbency material may be in any of a wide variety of geometric forms. As a general rule, it is preferred that the high-absorbency material be in the form of discrete particles. However, the high-absorbency material may also be in the form of fibres, flakes, rods, spheres, needles, spiral or semi-spiral, cubic, rod-like, polyhedral, or the like. Conglomerates of particles of high-absorbency material may also be used. The high-absorbency material may be present in the absorbent core in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

As used herein, "Substantially liquid impermeable" as used herein refers to a barrier (or wicking layer) that comprises a material that will substantially prevent the passage of liquids through it. **It** should be noted that the material can be breathable so as to permit the passage of gasses or vapors, but not of liquids, or the material can be configured so as to prevent the passage of liquids as well as gasses or vapors. The degree of impermeability of a material is typically measured by the hydrostatic head.

"Substantially absorbent particulate polymer material impermeable" as used herein refers to an edge barrier (or wicking layer) that comprises a material that will substantially prevent the passage of absorbent particulate polymer material through it.

"Integral" is used to refer to various portions of a single unitary element rather than separate structures bonded to or placed with or placed near one another.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" is intended to refer to the article when it is flattened into a plane or is substantially flattened into a plane and is used in contrast to when the article otherwise positioned, such as when the article is folded or shaped in or for use by a wearer.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

"Longitudinal" is a direction running parallel to the maximum linear dimension of the article.

The term "machine direction" or "MD" may be used herein to refer to the direction of material flow through a manufacturing process. In addition, relative placement and movement of material can be described as flowing in the machine direction through a process from upstream in the process to downstream in the process. The term "cross direction" or "CD" may be used herein to refer to a direction that is generally perpendicular to the machine direction.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. **In** general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "nonelastic" or "inelastic" refers to any material which does not fall within the definition of "elastic" above, typically by not comprising elastic materials such as elastic web materials described herein.

The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

"Proximal" and "Distal" refer respectively to the location of an element relatively near to or far from the longitudinal or lateral centerline of a structure (e.g., the proximal edge of a longitudinally extending element is located nearer to the longitudinal axis than the distal edge of the same element is located relative to the same longitudinal axis).

"Pulp fluff" or "fluff pulp" refers to a material made up of cellulose fibers. The fibers can be either natural or synthetic, or a combination thereof. The material is typically lightweight and has absorbent properties.

As used herein the term "sheet" or "sheet material" refers to woven materials, nonwoven webs, polymeric films, polymeric scrim-like materials, and polymeric foam sheeting.

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

By "stretch", it is meant that the material has the ability to extend beyond its original size in at least one dimension when subjected to a tensile force (i. e., tension) applied in the direction of that dimension, without breaking the material. An extension of for example 50% means that the material with an initial length of 100mm has reached a length of 150mm. "Stretch" may be unidirectional, bidirectional, or multi- directional. The specific "stretch" properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.

By "substantially", it is meant at least the majority of the structure referred to.

Use of the term "substrate" includes, but is not limited to, woven or nonwoven webs, porous films, ink permeable films, paper, composite structures, or the like.

Superabsorbent materials suitable for use in the present invention are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCl). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 60% by weight. Typically, the superabsorbent material, when present, will be included in an amount of about 5% to about 40% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

"Tension" includes a uniaxial force tending to cause the extension of a body or the balancing force within that body resisting the extension.

As used herein, the term "thermoplastic" is meant to describe a material that softens when exposed to heat and which substantially returns to its original condition when cooled to room temperature.

The term "topsheet" or "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet may be joined to portions of the backsheet, the absorbent core, the barrier leg cuffs and/or any other layers as is known to those of ordinay skill in the art. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction. "Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighbouring particles. Embodiments of the articles according to the disclosure will now be described. It is understood that technical features described in one or more embodiments maybe combined with one or more other embodiments without departing from the intention of the disclosure and without generalization therefrom.

### GENERAL DESCRIPTION OF ABSORBENT ARTICLE

According to the present disclosure, "Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn.

Disposable or reusable absorbent articles (1) herein, as exemplified in Fig. 1, comprise a liquid pervious topsheet (2), a liquid impermeable backsheet (4) joined to the topsheet (2), and an absorbent core (3) positioned and held at least partially between the topsheet (2) and the backsheet (4). The topsheet (2) is operatively permeable to the liquids that are intended to be held or stored by the absorbent article, and the backsheet (4) is substantially liquid impervious or otherwise operatively impermeable to the intended liquids. The disposable or reusable absorbent articles (1) further comprise a pair of barrier leg cuffs (5,5'), each barrier leg cuff (5,5') formed from a single web of material and comprising an inner cuff element (6,6') and an outer cuff element (7,7') and wherein the inner cuff element (6,6') comprises an inner folded edge (6f,6f') and one or more, preferably a plurality of, inner elastic elements (8,8'), wherein the inner elastic elements (8,8') are disposed proximate the inner folded edge (6f,6f'); and wherein the outer cuff element (7,7') comprises an outer folded edge (9,9') and from 1 to 5 outer elastic elements (10,10'); and wherein the outer elastic elements (10,10') are disposed proximate the outer folded edge (9,9') and the one or more outer elastic elements (10,10') are disposed at least partly between a first part (4f,4f') and a second part (4s) of said liquid impermeable backsheet (4).

The absorbent article (1) is generally constructed around a central chassis, which provides a structural foundation and functional elements for fluid management. The chassis may comprise a liquid permeable topsheet (2), a liquid impermeable backsheet (4), and an absorbent core (3). The topsheet (2) typically forms the innermost layer (i.e. a skin-facing or body-facing layer), designed for direct contact with the wearer's skin. Its primary function may be to rapidly acquire discharged fluids, such as urine or menstrual fluid, and transport them to the underlying absorbent core (4), while minimizing rewetting and maintaining a dry and comfortable surface against the skin. To achieve this, the topsheet (2) is generally selected to exhibit liquid permeability, softness, and non-irritation. Liquid permeability is achieved through various material properties and structural features, such as pore size and distribution designed for efficient fluid intake, or through capillary action, drawing fluid away from the surface. Surface treatments, in the form of hydrophilic finishes or surfactants, may also be applied to the topsheet (2) to enhance its wettability and liquid acquisition rate. One such preferred example is the use of a zone-treated topsheet with distinct regions of differing wettability. The topsheet (2) may be a nonwoven fabric (e.g., spunbond, meltblown, carded, hydroentangled), an apertured film, or a laminate. Nonwoven fabrics may be composed of synthetic fibers (e.g., polypropylene, polyethylene, polyester), natural fibers (e.g., cotton, cellulose), or combinations thereof. For example, the topsheet (2) may comprise a spunbond nonwoven (e.g. from PFN, 100 Wt% PP). The selection of fiber type and nonwoven construction influences the topsheet's softness, liquid handling properties, and structural integrity. For example, air-through bonded nonwovens are known for softness and loft, while spunbond nonwovens offer good strength and durability.

Preferably, the absorbent article (1) comprises a zone-treated topsheet (2) having distinct hydrophilic and hydrophobic regions generally to optimize fluid management and leakage prevention. Such zone-treated topsheet provides for a central hydrophilic zone interposed between two hydrophobic zones positioned proximal and/or adjacent to the barrier leg cuffs (5, 5'). This configuration in particular may help to address a specific challenge often observed with certain topsheet materials, such as those exhibiting enhanced loft, softness, or premium fluid acquisition, which can exhibit an increased propensity for lateral fluid migration. This migration can occur via capillary action within the topsheet structure, guiding fluid underneath the cuff attachment and towards the article's periphery, leading to leakage. By strategically incorporating hydrophobic zones adjacent to the cuff attachment areas, a further barrier is effectively created that impedes this lateral fluid transport, effectively mitigating the risk of side leakage.

The backsheet (4) generally forms the outermost layer (i.e. a garment-facing layer), preventing leakage of fluids from the absorbent core (3) and protecting clothing and bedding. Backsheets herein are preferably in the form of a film. Key characteristics of the backsheet (4) may include liquid impermeability and, optionally, breathability. Liquid impermeability may be achieved through a material that is substantially impermeable to liquids, preventing strike-through even under pressure. Suitable materials include thin plastic films (e.g., polyethylene), coated nonwovens, or laminates of films and nonwovens. The liquid impervious film layer may be made of polyethylene (PE). Preferably, the backsheet (4) is breathable, allowing water vapor to escape from the absorbent core (3) while maintaining liquid impermeability. This may enhance comfort and may reduce the risk of skin irritation by promoting airflow. Breathability can be achieved through microporous films or laminates. The backsheet material may be breathable so as to allow vapor to escape from the absorbent material, while still preventing liquids from passing there through. Examples of breathable backsheet materials are porous polymeric films, nonwoven laminates of spunbond and meltblown layers and laminates of porous polymeric films and nonwoven materials, in such laminates, the polymeric film can have the same or a different width than the nonwoven material.

The absorbent core (3) is generally positioned between the topsheet (2) and backsheet (4) and is the primary component responsible for absorbing and retaining fluids. The absorbent core (3) may be a multi-functional structure designed for efficient fluid management. The absorbent core (3) not only absorbs and stores fluids but may also play a role in distributing fluids and maintaining the integrity of the absorbent article (1). The absorbent core (3) may typically be enclosed within a core wrap (12,13), which is a layer of material (typically a nonwoven) that surrounds the absorbent material. The core wrap (12,13) generally prevents the absorbent material (e.g., SAP and cellulose fibers) from migrating out of the core and into other parts of the absorbent article (1) and helps to maintain the shape and structural integrity of the absorbent core (3), both when dry and when saturated with fluid. The core wrap material (12,13) may be a nonwoven fabric, a tissue, or a combination of materials, selected for their strength, flexibility, and fluid handling properties. Typically the basis weight of the core wrap layer(s) (12,13) is from 5 gsm to 50 gsm, preferably from 6 gsm to 35 gsm, more preferably from 8 gsm to 30 gsm. The absorbent material may be contained within the absorbent article (1) by the topsheet (2) and backsheet (4) only without the presence of a core wrap layer (12,13). The core wrap layer(s) (12,13) can be made of environmentally friendly material, preferably biodegradable and/or compostable material, selected from and not limited to polylactic acid (PLA), viscose, polyhydroxyalcanoates (PHA), bio-derived polyethylene, (bioPE), low density polyethylene (LDPE), poly-3-hydroxybutyrate (PHB) or polycaprolactone (PCL).

The absorbent material within the absorbent core (3) is generally responsible for absorbing and retaining fluids. This material may be selected from a variety of suitable options, including superabsorbent polymers (SAPs) and cellulose fibers. SAPs are more generally cross-linked polymers capable of absorbing many times their weight in liquid. They may be strategically distributed throughout the absorbent core (3) to maximize fluid retention. Different types of SAP may be selected based on their absorption rate, retention capacity, and gel strength. Cellulose fibers, such as wood pulp fluff, are preferably used in conjunction with SAPs. The cellulose fibers may provide bulk, structure, fluid distribution, and cost-effectiveness. The absorbent material herein preferably comprises a mixture of superabsorbent particles with intermixed and dispersed cellulose fibers therein, for high absorbent capacity and good fluid distribution properties.

The absorbent core (3) may further comprise an acquisition and distribution layer, ADL, (17) positioned between the liquid permeable topsheet (2) and the absorbent core (3). The ADL (17) is generally designed to rapidly acquire discharged bodily fluids, such as urine, and then distribute these fluids to the absorbent core (3) for retention. This may help to prevent pooling and rewetting of the topsheet (2), maintaining a dry and comfortable surface against the wearer's skin. The ADL may typically be less hydrophilic than the absorbent layer and may typically be made from a nonwoven, such as a spunbond nonwoven, a carded nonwoven, a through-air bonded, thermo-pressure bonded, or chemically bonded carded nonwoven.

The absorbent core (3) may comprise one or more channels that are substantially free of absorbent material. These channels generally facilitate fluid distribution within the absorbent core (3) and prevent bunching or clumping of the absorbent material when the core is wetted. The channels may be embossed but are preferably formed by bonding top and bottom layers of the core wrap (12,13) together at attachment areas substantially corresponding to the channel shape(s). Advantageously, the addition of one or more channels to the absorbent core of the present disclosure may enhance the ability of the barrier leg cuffs of the present disclosure to prevent leakage. An optimal liquid distribution, as an effect of the one or more channel(s), may prolong the time it takes for the barrier leg cuffs to come into contact with exudates. Typically the channels have a width generally greater than 1 mm, preferably from 5 mm to 50 mm, more preferably from 6 mm to 40 mm, more preferably from 8 mm to 30 mm, even more preferably from greater than 8 mm to less than 25 mm. The one or more channels are arranged within the inner periphery of the absorbent core, i.e. the inner, or interior, space defined by the periphery of the absorbent core, such that each of the channel(s) is bounded, or surrounded, by absorbent material. Additionally, the length of the channels may be from 10 % to 95 % of the length of the absorbent core (3) so that again the one or more channels are bounded by absorbent material.

In addition to the components described above, the absorbent article (1) may incorporate one or more further features designed to enhance its fit, comfort, and functionality. For absorbent articles (1) configured as open diapers, elastic ears may be included at the rear corners of the chassis. These elastic ears provide improved fit and conformability around the wearer's waist and contribute to a more secure and comfortable fit. A fastening system may also be included with open diaper configurations, which enables the securement of the absorbent article (1) around the wearer's body. The fastening system may comprise adhesive tapes, hook-and-loop fasteners, or other suitable mechanical closures. In embodiments utilizing hook-and-loop fasteners, a landing zone or receiving area may be provided on the outer surface of the front portion of the absorbent article (1) to facilitate secure and adjustable fastening. For absorbent articles (1) configured as pull-on pants, elastic front and back waist belts are incorporated into the design. These elastic waist belts are seamed together at the side edges of the absorbent article (1), forming a closed, pant-like configuration. The elastic waist belts provide a snug and comfortable fit around the wearer's waist, allowing the absorbent article (1) to be easily pulled up and down like regular underwear.

### THE BARRIER LEG CUFFS

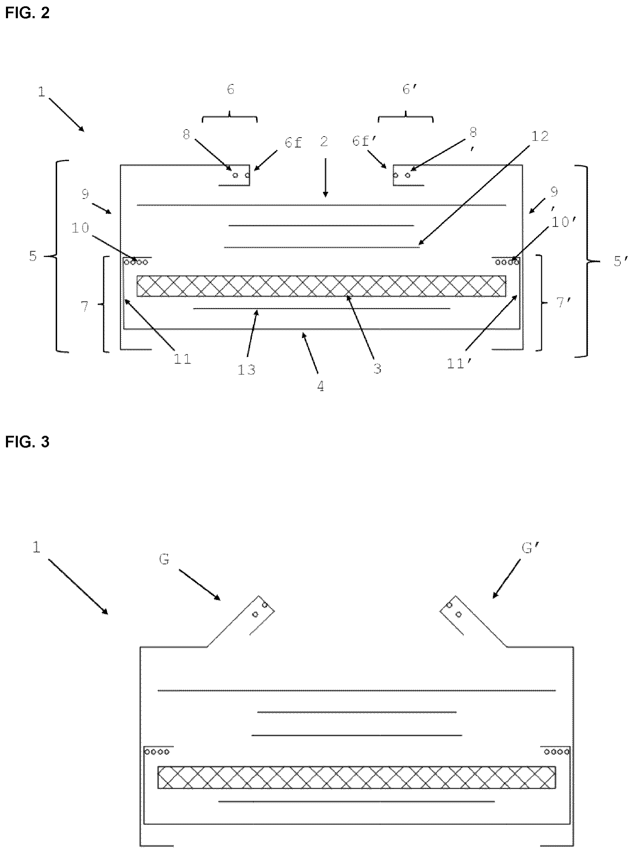

The barrier leg cuffs (5,5') of the absorbent article (1) of the present disclosure, as exemplified in Fig.2, are formed from a single web of material for efficient manufacturing and cost-effectiveness. Each barrier leg cuff (5,5') includes an inner cuff element (6,6') and an outer cuff element (7,7'). The inner cuff element (6,6') is designed to create a close and comfortable seal against the wearer's skin, preventing fluids from escaping the absorbent article (1). The inner cuff element (6,6') comprises an inner folded edge (6f,6f') providing a soft and smooth surface against the wearer's skin, minimizing the risk of irritation. One or more inner elastic elements (8,8') are disposed proximate the inner folded edge (6f,6f') to provide a contractive force that gently urges the inner cuff element (6,6') towards the wearer's skin. The outer cuff element (7,7') is designed to provide additional containment and helps to maintain the overall shape and structure of the barrier leg cuff (5,5'). The outer cuff element comprises an outer folded edge (9,9') to provide a soft and smooth surface, minimizing the risk of irritation. From 1 to 5 outer elastic elements (10,10') are disposed proximate the outer folded edge (9,9') and contribute to the overall contractive force of the barrier leg cuff (5,5'). To ensure reliable adhesion and positioning of the inner elastic elements (8,8'), the inner elastic elements (8, 8') may be strand-coated with adhesive. This strand-coating technique generally applies a thin, uniform layer of adhesive to the elastic elements, providing a strong and durable bond between the elastic elements and the surrounding nonwoven layers, yet permits to minimize the amount of glue by surface area and hence improving on the above mentioned benefits.

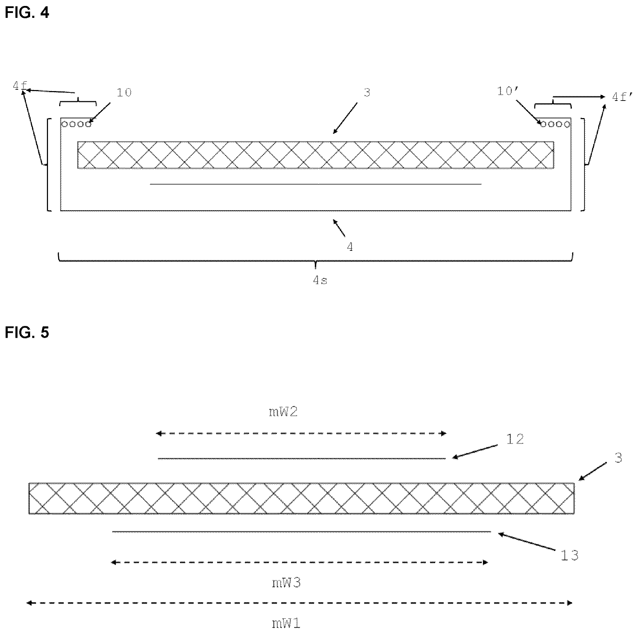

In a preferred embodiment, as exemplified in Fig.2 and Fig.4, the outer elastic elements (10,10') are disposed at least partly between a first part (4f,4f') and a second part (4s) of the liquid impermeable backsheet (4). This particular configuration of the liquid impermeable backsheet (4), with the elastic elements positioned between its layers, may provide enhanced functionality, performance and minimal leakage. To ensure reliable adhesion and positioning of the outer elastic elements (10,10'), the outer elastic elements (10, 10') may be strand-coated with adhesive. This strand-coating technique generally applies a thin, uniform layer of adhesive to the elastic elements, providing a strong and durable bond between the elastic elements and the surrounding nonwoven layers, yet permits to minimize the amount of glue by surface area and hence improving on the above mentioned benefits.

In a first example according to the preferred embodiment, the inner cuff element (6,6') is configured to form an up-standing gather (G,G'), as exemplified in Fig.3. This up-standing gather acts as a physical barrier, and may further enhance the containment of bodily fluids within the absorbent article. The up-standing gather helps to channel fluids towards the absorbent core and prevents them from escaping through the leg openings. This feature is particularly effective in managing surges of fluid and preventing side leakage.

In a second example according to any of the preferred embodiment and first example, the inner cuff element (6,6') may be configured to be substantially T-shaped, when viewed in a cross-section taken about an axis substantially perpendicular to the longitudinal center axis (y-y). This T-shape may provide a distinct advantage in conforming to the wearer's body. The vertical portion of the "T" provides height and stand-up rigidity for containing fluids, while the horizontal portion of the "T" may provide a wider, softer contact area against the skin, minimizing pressure points and enhancing wearer comfort. This configuration may ensure that the inner cuff element effectively acts as a fluid barrier without causing undue irritation or discomfort.

In a third example, as exemplified in Fig.2, according to any of the preferred embodiment and previous examples, the liquid impermeable backsheet (4) comprises an outer folded edge (11,11') such that the first part (4f,4f') of said liquid impermeable backsheet (4) is folded laterally inward towards the longitudinal centerline (y-y) in overlapping configuration with said second part (4s) of said liquid impermeable backsheet(4). This fold creates a double-layered section in the liquid impermeable backsheet (4), and may thereby provide increased resistance to fluid penetration and physical stress. The overlapping configuration may ensure that any fluid that might breach the first part (4f,4f') is contained by the second part (4s), further minimizing the risk of leakage. Advantageously, said configuration may enhance the structural integrity and leakage prevention capabilities of the absorbent article.

In a preferred embodiment of said third example, as exemplified in Fig.2, the outer elastic elements (10,10') are disposed proximal the outer folded edge (11,11') of said liquid impermeable backsheet (4). By positioning the outer elastic elements (10,10') near the folded edge (11,11') of the liquid impermeable backsheet (4), the contractive force is concentrated in this critical area. This arrangement enhances the seal created by the barrier leg cuffs (5,5') against the wearer's body, may prevent fluids from escaping through the leg openings. The proximity of the elastic elements to the folded edge may ensure that the edge remains closely conformed to the wearer's body, even during movement, which may provide a secure and reliable barrier against leakage.

In a fourth example, as exemplified in Fig.2, according to any of the preferred embodiment and previous examples, the absorbent core (3) comprises 2 laterally opposed longitudinal side edges (3s,3s'), a transversal front edge (3f) and a transversal back edge (3b) and the first part (4f,4f') of said liquid impermeable backsheet (4) extends along the entire thickness of the absorbent core (3). This configuration may ensure that the liquid impermeable backsheet fully protects the absorbent core (3) from fluid strike-through, preventing leakage and maintaining a dry outer surface. The liquid impermeable backsheet (4) may thereby provide a comprehensive barrier that encompasses all layers of the core, maximizing its containment capabilities.

In a fifth example, as exemplified in Fig.2, according to any of the preferred embodiment and previous examples, the absorbent core (3) comprises a top core wrap layer (12) and a bottom core wrap layer (13) and absorbent material arranged partially between said top core wrap layer (12) and said bottom core wrap layer (13). This arrangement creates a structured absorbent core with distinct layers for different functions and ensures containment of the absorbent material within said absorbent core.

In a preferred embodiment of said fifth example, as exemplified in Fig.5, the absorbent core (3) has a maximum width mW1, the top core wrap layer (12) has a maximum width mW2 and the bottom core wrap layer (13) has a maximum width mW3, wherein mW1 is greater than mW2 and/or mW3. The maximum widths mW1, mW2 and mW3 are measured in fully extended state of the absorbent article (1) and parallel to the transverse center axis (x-x). This configuration may provide an optimal balance between structural integrity of the absorbent core and swelling capacity of the absorbent core thereby optimally making use of the available liquid absorption capacity.

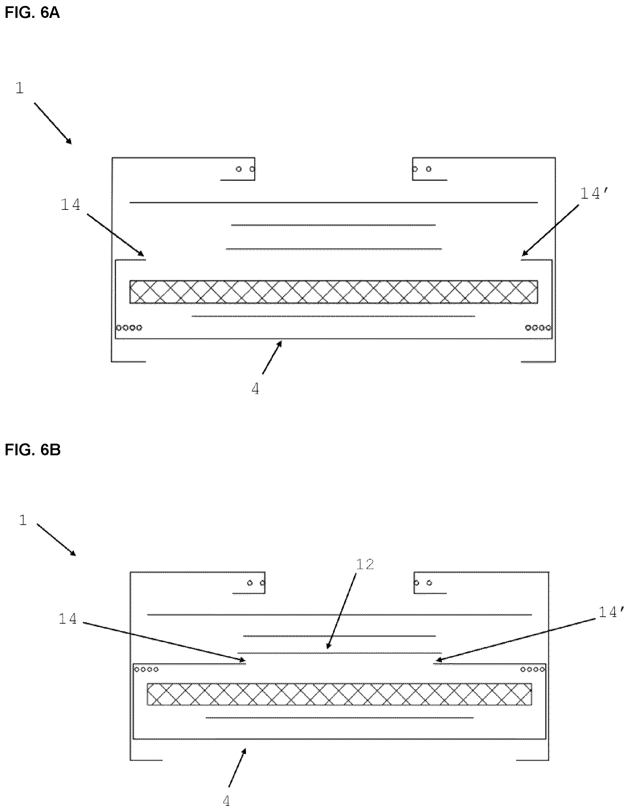

In a first embodiment of said preferred embodiment of said fifth example, as exemplified in Fig.6A, the first part (4f,4f') of the liquid impermeable backsheet (4) is attached to the wearer-facing surface of the absorbent core (3). This securement may provide stability to the structure and may prevent the backsheet from shifting or bunching during use. This attachment ensures that the backsheet remains in close proximity to the absorbent core, maximizing its effectiveness in preventing fluid strike-through. Preferably, the first part (4f,4f') of the liquid impermeable backsheet (4) comprises an end edge (14,14') wherein said end edge (14,14') is attached to the wearer-facing surface of the absorbent core (3). This direct connection between the liquid impermeable backsheet and the absorbent may provide a secure and reliable bond that resists separation or shifting, even during vigorous activity.

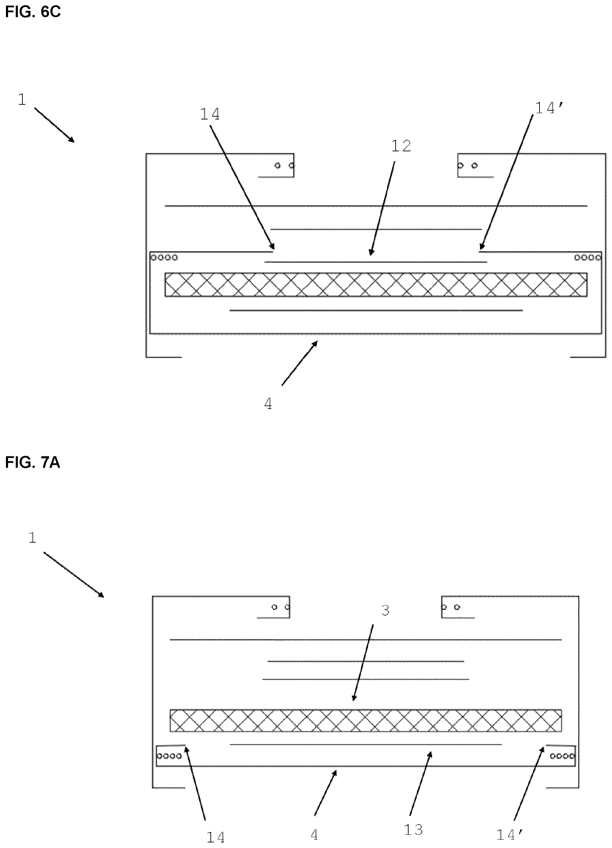

In a second embodiment of said preferred embodiment of said fifth example, as exemplified in Fig.6B and 6C, the first part (4f,4f') of the liquid impermeable backsheet (4) comprises an end edge (14,14'), and this end edge (14,14') is attached to the wearer-facing side and/or the garment-facing side of the top core wrap layer (12). By attaching the end edge (14,14') of the first part (4f,4f') of the liquid impermeable backsheet (4) to the top core wrap layer (12), the construction may ensure that the liquid impermeable backsheet (4) remains securely anchored to the absorbent core (3) assembly, even when subjected to stresses and strains during use. This configuration may also improve the overall integrity and durability of the absorbent article, minimizing the risk of delamination or separation of the backsheet from the core.

In a third embodiment according to said preferred embodiment of said fifth example, as exemplified in Fig.7A, the first part (4f,4f') of the liquid impermeable backsheet (4) is attached to the garment-facing surface of the absorbent core (3). This securement method may provide a robust connection between the liquid impermeable backsheet (4) and the absorbent core (3). Preferably, the first part (4f,4f') of the liquid impermeable backsheet (4) comprises an end edge (14,14'), and this end edge (14,14') is attached to the garment-facing surface of the absorbent core (3). By attaching the end edge of the first part of the backsheet to the garment-facing surface of the absorbent core, the absorbent article may achieve a reliable and durable construction that resists separation and maintains its structural integrity throughout use.

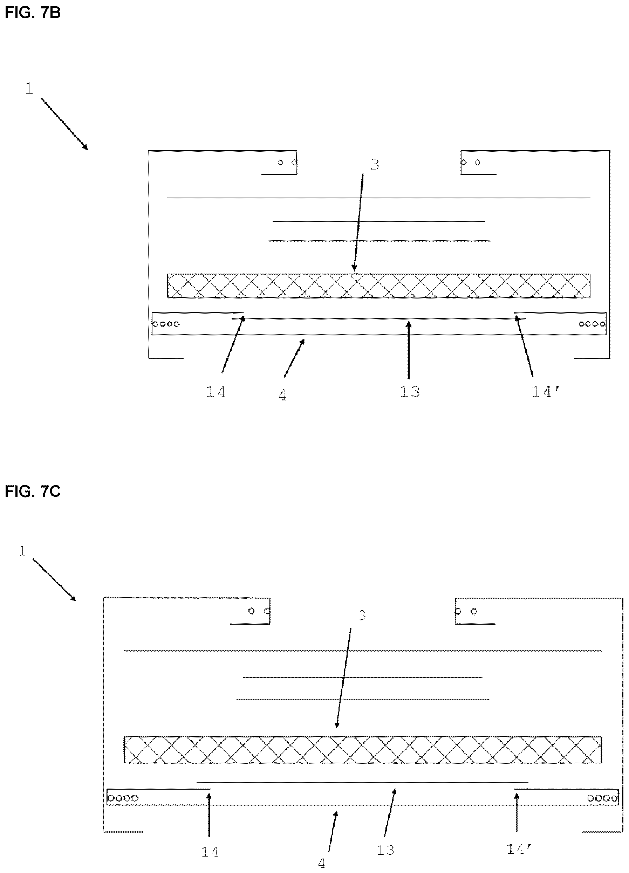

In a fourth embodiment of said preferred embodiment of said fifth example, as exemplified in Fig.7B and 7C, the first part (4f,4f') of the liquid impermeable backsheet (4) comprises an end edge (14,14'), and this end edge (14,14') is attached to the wearer-facing surface of the bottom core wrap (13) layer and/or to the garment-facing surface of the bottom core wrap layer (13). This attachment strategy may further secure the first part of the backsheet, providing a robust and reliable construction that resists separation and maintains structural integrity.

In an alternative embodiment of said fifth example, the top core wrap layer (12) and the bottom core wrap layer (13) may be a single sheet that is folded onto itself to contain the absorbent material therein or may comprise 2 distinct sheets that are bonded to each other at least at the peripheral edges thereof to sandwich the absorbent material therein. Several core wrap constructions are possible such as G-fold, C-fold or sandwich wraps.

In an embodiment of said alternative embodiment of said fifth example, the first part (4f,4f') of the liquid impermeable backsheet (4) comprises an end edge (14,14'), and this end edge (14,14') is attached to the wearer-facing side of the top core wrap layer (12) and/or bottom core wrap layer (13). By attaching the end edge (14,14') of the first part (4f,4f') of the liquid impermeable backsheet (4) to the wearer-facing side of the top core wrap layer (12) and/or bottom core wrap layer (13), the construction may ensure that the liquid impermeable backsheet (4) remains securely anchored to the absorbent core (3) assembly, even when subjected to stresses and strains during use. This configuration may also improve the overall integrity and durability of the absorbent article, minimizing the risk of delamination or separation of the backsheet from the core.

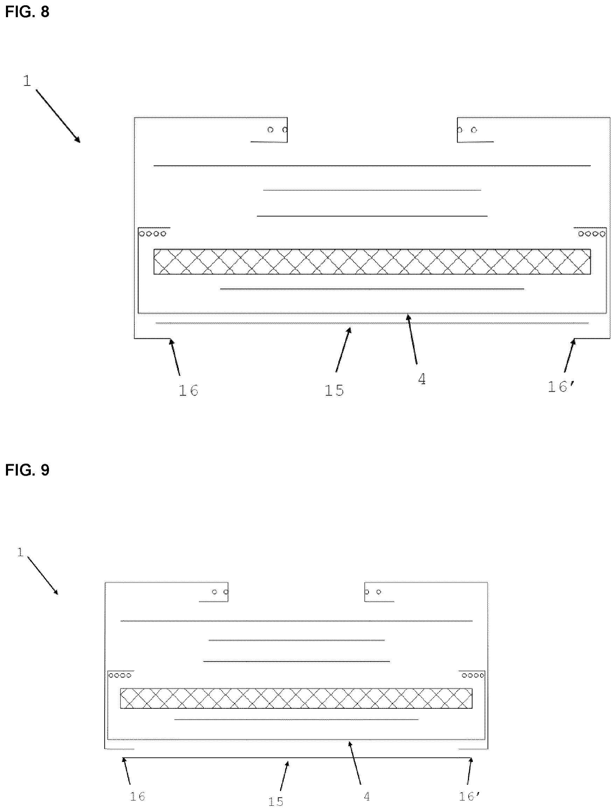

In a sixth example according to any of the previous examples and embodiments, as exemplified in Fig.8, the absorbent article may also comprise a NW backsheet (15) at least partially attached to the garment-facing surface of said liquid impermeable backsheet (4). The NW backsheet (15) may provide a soft, cloth-like feel to the exterior of the absorbent article (1), enhancing its comfort and appeal to the wearer.

In a first embodiment according to said sixth example, said NW backsheet (15) is attached to the liquid impermeable backsheet (4) by ultrasonic bonding. Advantageously, a durable and secure attachment might be achieved without the need for adhesives.

In an alternative embodiment of said first embodiment according to said sixth example, the NW backsheet (15) may be attached to the liquid impermeable backsheet by adhesive. Preferably, the NW backsheet (15) is attached to the liquid impermeable backsheet (4) by means of a plurality of longitudinal adhesive strips parallel to one another and spaced apart from one another by adhesive-free longitudinal strips. Additionally, transverse adhesive strips may be provided at the front end region and/or back end region of the NW backsheet (15). Advantageously, such configuration may allow to absorb shear forces and compression in the transverse direction. Further advantageously, the alternating arrangement of longitudinal adhesive strips and adhesive-free longitudinal strips avoids stiffening and rather provides flexibility in the transverse direction. Even further advantages are reduced adhesive usage and improved breathability supported by the formation of channel-like or tunnel-like regions which extend in the longitudinal direction.

In a second embodiment according to any of said sixth example, first embodiment and alternative embodiment of said sixth example, as exemplified in Fig.8, said single web of material comprises an end edge (16,16') and said end edge (16,16') is attached to the garment-facing surface of the NW backsheet (15). This configuration may ensure that the leg cuffs are securely anchored to the outer layers of the absorbent article, preventing them from shifting or detaching during use.

In a third embodiment according to any of said sixth example and first embodiment of said sixth example, as exemplified in Fig.8 and Fig.9, said single web of material comprises an end edge (16,16') and said end edge (16,16') is attached to the wearer-facing surface of the NW backsheet (15). This configuration may ensure that the leg cuffs are securely anchored to the inner layers of the absorbent article, preventing them from shifting or detaching during use.

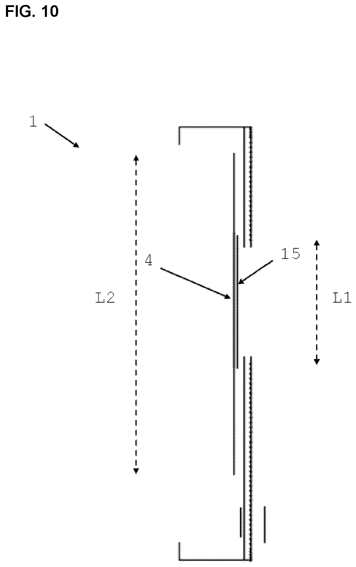

In a fourth embodiment according to any of said sixth example and its embodiments, as exemplified in Fig.10, said absorbent article (1) comprises a transverse center axis (x-x) perpendicular to the longitudinal center axis (y-y) and wherein said NW backsheet (15) has a length L1 parallel to the longitudinal center axis (y-y) and said liquid impermeable backsheet (4) has a length L2 parallel to the longitudinal center axis (y-y), wherein L1 is less than L2. This dimensional relationship may optimize the coverage and functionality of the NW backsheet, providing a balance of softness, breathability, and leakage protection with an optimized raw material usage.

## Claims

1. An absorbent article (1) comprising:
a liquid permeable topsheet (2);
a liquid impermeable backsheet (4);
an absorbent core (3) disposed at least partially between the topsheet (2) and the backsheet (4);
and a pair of barrier leg cuffs (5,5'), each barrier leg cuff (5,5') formed from a single web of material and comprising an inner cuff element (6,6') and an outer cuff element (7,7');
wherein the inner cuff element (6,6') comprises an inner folded edge (6f,6f) and one or more, preferably a plurality of, inner elastic elements (8,8'), wherein the inner elastic elements (8,8') are disposed proximate the inner folded edge (6f,6f'); and wherein the outer cuff element (7,7') comprises an outer folded edge (9,9') and from 1 to 5 outer elastic elements (10,10');
**characterised in that** the outer elastic elements (10,10') are disposed proximate the outer folded edge (9,9'); and **in that** the one or more outer elastic elements (10,10') are disposed at least partly between a first part (4f,4f') and a second part (4s) of said liquid impermeable backsheet (4).

2. An absorbent article (1) according to Claim 1, wherein the inner cuff element (6,6') forms an up-standing gather (G,G').

3. An absorbent article (1) according to any of Claim 1 to 2, wherein said absorbent article (1) comprises a longitudinal center axis (y-y) and wherein the inner cuff element (6,6') is substantially T-shaped, preferably when viewed from a section taken about an axis being substantially perpendicular to the longitudinal center axis (y-y).

4. An absorbent article (1) according to any of the preceding Claims, wherein said liquid impermeable backsheet (4) comprises an outer folded edge (11,11') such that the first part (4f,4f') of said backsheet (4) is folded laterally inward towards the longitudinal centerline (y-y) in overlapping configuration with said second part (4s) of said backsheet (4).

5. An absorbent article (1) according to Claim 4, wherein said outer elastic elements (10,10') are disposed proximal the outer folded edge (11,11') of said liquid impermeable backsheet (4).

6. An absorbent article () according to any of the preceding claims, wherein said absorbent core (3) comprises 2 laterally opposed longitudinal side edges (3s,3s'), a transversal front edge (3f) and a transversal back edge (3b) and wherein said first part (4f,4f') of said liquid impermeable backsheet (4) extends along the entire thickness of the absorbent core (3).

7. An absorbent article (1) according to any of the preceding claims, wherein said absorbent core (3) comprises a top core wrap layer (12) and a bottom core wrap layer (13) and absorbent material arranged partially between said top core wrap layer (12) and said bottom core wrap layer (13).

8. An absorbent article (1) according to Claim 7, wherein the absorbent core (3) has a maximum width (mW1), the top core wrap layer (12) has a maximum width (mW2) and the bottom core wrap layer (13) has a maximum width (mW3), wherein mW1 is greater than mW2 and/or mW3.

9. An absorbent article (1) according to Claim 8 , wherein said first part (4f,4f') of said liquid impermeable backsheet (4) is attached to the wearer-facing surface of the absorbent core (3).

10. An absorbent article (1) according to Claim 9, wherein said first part (4f,4f') of said liquid impermeable backsheet (4) comprises an end edge (14,14') and wherein said end edge (14,14') is attached to the wearer-facing surface of the absorbent core (3).

11. An absorbent article (1) according to any of Claims 8 to 9, wherein said first part (4f,4f') of said liquid impermeable backsheet (4) comprises an end edge (14,14') and wherein said end edge (14,14') is attached to the wearer-facing side and/or the garment-facing side of top core wrap layer (12).

12. An absorbent article (1) according to Claim 8, wherein said first part (4f,4f') of said liquid impermeable backsheet (4) is attached to the garment-facing surface of the absorbent core (3).

13. An absorbent article (1) according to Claim 12, wherein said first part (4f,4f') of said liquid impermeable backsheet (4) comprises an end edge (14,14') and wherein said end edge (14,14') is attached to the garment-facing surface of the absorbent core (3).

14. An absorbent article (1) according to any of Claims 8 or 12, wherein said first part (4f,4f') of said liquid impermeable backsheet (4) comprises an end edge (14,14') and wherein said end edge (14,14') is attached to the wearer-facing surface of the bottom core wrap layer (13) and/or to the garment-facing surface of the bottom core wrap layer (13).

15. An absorbent article (1) according to any of the preceding Claims, wherein said absorbent article (1) further comprises a NW backsheet (15) at least partially attached to the garment-facing surface of said liquid impermeable backsheet (4).

16. An absorbent article (1) according to Claim 15, wherein said NW backsheet (15) is attached to the liquid impermeable backsheet (4) by ultrasonic bonding.

17. An absorbent article (1) according to any of claims 15 to 16, wherein said single web of material comprises an end edge (16,16') and said end edge (16,16') is attached to the garment-facing surface of the NW backsheet (15).

18. An absorbent article (1) according to any of Claims 15 to 16 wherein said single web of material comprises an end edge (16,16') and said end edge (16,16') is attached to the wearer-facing surface of the NW backsheet (15).

19. An absorbent article (1) according to any of Claims 15 to 18 wherein said article (1) comprises a transverse center axis (x-x) perpendicular to the longitudinal center axis (y-y) and wherein said NW backsheet (15) has a length L1 parallel to the longitudinal center axis (y-y) and said liquid impermeable backsheet (4) has a length L2 parallel to the longitudinal center axis (y-y), wherein L1 is less than L2.
